# EUROPEAN PATENT SPECIFICATION

(11) **EP 2 063 812 B1**
(45) Date of publication and mention of the grant of the patent: **24.11.2010**
(21) Application number: 07789274.3
(22) Date of filing: 21.08.2007
(51) Int. Cl.: A61F 2/06

(54) **IMPROVEMENTS IN AND RELATING TO MEDICAL DEVICES**
VERBESSERUNG VON UND IM ZUSAMMENHANG MIT MEDIZINPRODUKTEN
AMÉLIORATIONS APPORTÉES À DES APPAREILS MÉDICAUX OU AFFÉRENTES À CES DERNIERS

(30) Priority: 23.08.2006 GB 0616738
(43) Date of publication of application: 03.06.2009
(73) Proprietor: AME HEALTH LIMITED, Shirley Surrey CRO 8UJ (GB)
(72) Inventor: BARKER, Stephen, George, Edward, London, W1T4TP (GB); BROWN, Robert, Albert, Brockley Hill, London HA7 4LP (GB)
(74) Representative: Price, Nigel John King
(86) International application number: PCT/GB2007/003174
(87) International publication number: WO 2008/023160

(56) References cited:
- EP-A- 1 634 609
- WO-A-02/05731
- WO-A-95/10989
- WO-A-03/092471
- WO-A-2006/066223
- WO-A1-2004/103187
- US-A- 5 693 088

## Description

This invention relates to endoluminal prostheses for use in the treatment of dilations such as abdominal aortic aneurysms. The prostheses may find more general application, for example being used in the manner of stents following dilation of a vessel as a result of a balloon angioplasty procedure.

An abdominal aortic aneurysm (AAA) is a localised weakening of the walls of the aorta, which causes the aorta to expand or balloon. If this is left untreated the aneurysm can burst with fatal consequences.

An established treatment procedure is to open up the patient's abdomen, open the aneurysm by cutting into it and then to stitch in a graft, such as a Dacron (Trade Mark) tube, to replace the damaged section of aorta. More specifically, for repair of an AAA, a tube, or Y-shaped trouser graft, is commonly sewn in to the aorta during an open operation. This operation necessitates a mid-line abdominal incision, from the xiphisternum to the pubis, with exposure of the retro-peritoneal AAA. After clamping of vessels above and below the aneurysm, the sac would be opened and the graft inlaid. A proximal anastamosis would be fashioned, to provide a blood-tight seal, with a similar procedure done distally, either at the lowermost aorta, or in to the iliac vessels. The patient, post-procedure, would tend to go to the Intensive Therapy Unit for one to two days and then to stay on a ward for a further 10-14 days. Complete recovery would often take 3-6 months.

Because the surgeon has good access he/she can fix the opposite ends of the graft to the vessels in a fluid-tight manner. However, the highly invasive nature of open surgery of this sort, together with its long patient total recovery times, is undesirable.

There has been a move over the last 4-5 years to conducting this procedure less invasively, by using the endoluminal route to repair AAA. Devices exist that allow endoluminal graft placement from the region of the femoral artery which, although usually exposed, can be punctured percutaneously in selected cases. Usually, modular grafts are then delivered in retrograde fashion, to be sited across the aneurysmal sac, with the proximal part of the graft located in the aneurysm neck. The graft, bonded to an underlying metal stent is then deployed from within a delivery sheath, under fluoroscopic control. Once the stent is in place an angioplasty balloon catheter positioned internally of it can be expanded radially outwardly so as to expand the prosthesis into contact with the inner lumen of the aorta. More recently, memory-shaped materials such as Nitinol have been used for the stent, which materials have a memory and spring out to their original shape upon deployment from the delivery instrument, thereby avoiding the need actively to expand the stent using a balloon catheter. Such self-expanding devices are commonly provided on their opposite ends with a series of hooks which penetrate into the wall of the aorta to hold the device in place. To try to achieve a good seal between the graft and the internal diameter of the aortic neck (of the aneurysm) the graft is usually 'oversized'. For example, a 34mm diameter graft might be used to fit a 30mm diameter neck. Once deployed, the distal end(s) are sited and the femoral wound closed. Checks are made continuously to make sure the graft is sited appropriately. If all has gone well, the endoluminal approach to repair of AAA means a far lesser 'assault' on the patient; meaning no use of Intensive Therapy Unit facilities, a short hospital stay of 3-4 days only, with complete recovery in perhaps 3-6 weeks (to allow for the groin wounds to heal). One disadvantage, however, is the continual radiological follow-up the patients must undergo. The procedure, if it goes well, is cost effective.

A persistent problem with the less invasive endoluminal procedures is poor sealing between the prosthesis and the patient's aorta at its opposite ends, particularly at the proximal end (i.e. the upstream end as regards blood flow, which end is most proximal to the heart). If there is not good sealing between the prosthesis and the aortic or iliac vessel walls blood can leak into the sac of the aneurysm, causing the aneurysm to keep growing with the risk of eventual fatal rupture. This leakage is known generally as "endoleak", or more specifically "primary endoleak". It is thought that the incidence of endoleak in endolumenal AAA graft placement procedures is often as high as 40%. Prevention is through careful graft 'oversizing', correct assessment of aortic wall anatomy and correct graft placement. Treatment is by further covered stent placement (usually balloon-expandable stents) across the neck portion of graft (aorta), or by waiting to see if the leak will self-close (thrombose). Additional graft placement(s), and additional admissions and use of catheters, etc. significantly increases costs, making the whole procedure cost ineffective.

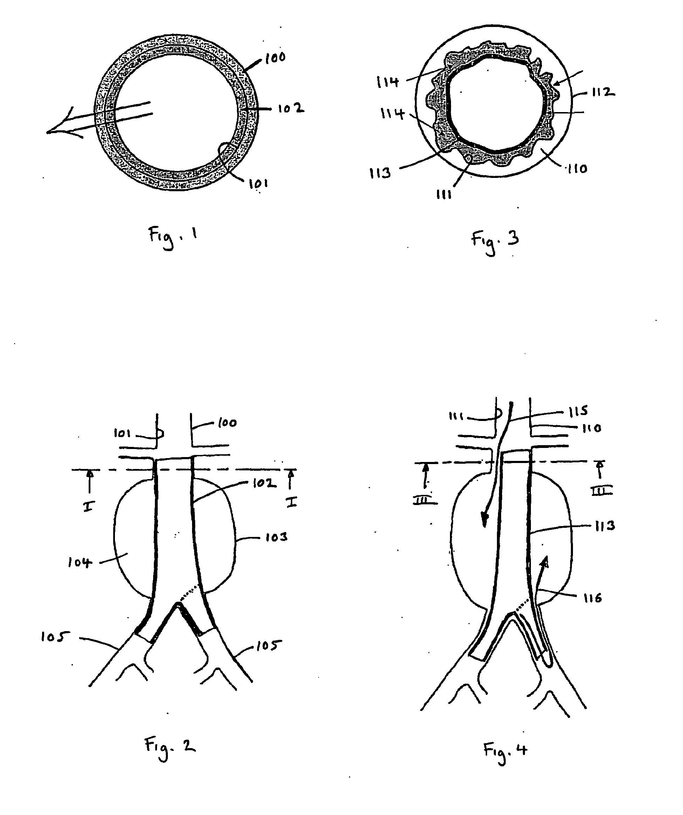

Primary endoleak occurs due to the poor fit of an aortic graft in an aorta. By way of explanation Figure 1 shows, in axial cross-section, the aorta 100 of a young and/or healthy individual. As can be seen, the cross-sectional shape of the aorta is circular. In addition to being cylindrical, the inner wall 101 of the aorta 100 is also fairly soft. This makes it easy for the external surface of a cylindrical graft 102 to form a snug fit with the inner wall 101, as shown. Particularly if the graft 102 is a self-expanding graft, made of a memory-shape material such as Nitinol, and is oversized, it will be appreciated that the external cylindrical surface of the graft 102 will be biased into firm and effective sealing contact with the inner wall 101 of the aorta, as denoted by the large arrow in the drawing.

Figure 2 shows, in longitudinal cross-section, a Y-shaped trouser graft 102 placed in the aorta 100, with its Y-shaped legs placed in the iliac vessels 104. The main cylindrical body of the graft 102 is shown positioned across the sac 104 of an abdominal aortic aneurysm 103. By achieving a good fit at the opposite ends of the graft 102 against the inner walls of the aorta 100 and iliac vessels 105 a good seal can be achieved at both ends of the graft 102. As a result, the blood flow along the aorta and iliac vessels is isolated from the blood in the sac 104 of the aneurysm 103 and primary endoleak is avoided.

In contrast, the aorta of an elderly and/or unhealthy individual is often neither generally cylindrical nor soft. The views shown in Figures 3 and 4 are generally similar to those shown in Figures 1 and 2 respectively, except that the aorta 110 pictured in Figures 3 and 4 is abnormal, having an irregular inner wall 111. Although the outer wall 112 of the aorta 110 is generally cylindrical, the inner wall 111 is commonly a mixture of heavy deposits of hard calcium, in conjunction with soft regions of fatty, cholesterol-rich deposit. The abnormal, non-cylindrical inner wall 111 presented to the external surface of the graft 113 is thus very difficult for the graft 113 to seal against. Not only is the graft 113 distorted by the abnormal, non-cylindrical nature of the inner wall 111 of the aorta 110, but the graft 113 is unable to form good sealing contact with the inner wall 111 around its full circumference. Although the gaps between the exterior of the graft 113 and the inner wall 111 of the aorta 110 shown in Figure 3 have been greatly exaggerated for reasons of clarity, it can be seen that fissures present in the inner wall 111 lead to gaps 114 between the graft 113 and the inner wall 111. As is shown schematically in Figure 4, these gaps 114 form possible sites for endoleak between the exterior of the graft 113 and the inner wall 111 of the aorta 110. In Figure 4 arrow 115 shows, schematically, a proximal route for primary endoleak. Arrow 116 shows a distal route for primary endoleak.

There is thus a need for an improved endoluminal prosthesis for use in the treatment of dilations including an improved endoluminal aortic aneurysm prosthesis to reduce the incidence of primary endoleak.

US patent application publication number US 2003/0074058 (published 17 April 2003) discloses a tubular prosthesis in which the prosthesis has an inner tube and an outer tube, which tubes may be sealed together at their ends. The inner tube is impervious and the outer tube has at least a pervious portion. An occluding fluid is, in use, conveyed through a fluid conduit into a pocket between the inner and outer members so as to cause portions of the outer member to expand and to seal against the wall of a blood vessel to prevent Type I endoleaks.

International patent publication number WO 03/003945 (published 16 January 2003) discloses an implant for use in the treatment of aortic aneurysms. The implant comprises a graft having a hem defining an interior space. Enclosed within this space is an absorbent cord. This cord expands as it comes into contact with body fluids. The expansion due to the absorbed fluids forms a seal closely following the irregular shape of the body lumen and improves fixation at the junction of the body lumen and the implant. Document WO 97/09009 discloses an expandable tubular member having a covering which swells in contact with blood and covers large-scale irregularities in the blood vessel way.

According to a first aspect of the present invention there is provided an endoluminal prosthesis, as appended in claim 1.

In a first example of the present invention this endoluminal prosthesis can be used in the treatment of a dilation in a body vessel, such as the aorta. The prosthesis comprises a tubular member that is radially expandable and which has proximal and distal ends, as well as an inner lumen and an outer surface. This tubular member also is provided on its outer surface, at least in the region of its proximal and distal ends, with a covering. This covering comprises first and second layers of material. The first and second layers are interleaved and extend generally spirally around the outer surface of the tubular member. The first layer comprises an agent that promotes swelling of the covering on exposure of the covering to contact with body fluid. The second layer comprises a space-filling material. The prosthesis may be configured so that, when delivered into the body vessel, the tubular member can self-expand or be expanded radially, with the covering swelling on the tubular member. The intention is for this expansion and swelling to force the exterior of the swollen covering into intimate sealing contact with the surrounding inner wall of the vessel. Where the dilation is an aneurysm, and the vessel is a blood vessel and the body fluid is blood, it is envisaged that primary endoleak will be substantially or completely avoided.

The space-filling material may be generally sponge-like, for example comprising collagen, gelatin or the like. It may also be advantageous for the space-filling material to be loaded with a drug or medicament, which might elute from the prosthesis following prosthesis delivery.

Instead of simply being provided in the region of the proximal and distal ends of the tubular member the covering may extend along substantially the complete length of the outer surface of the tubular member.

According to another example of the present invention the endoluminal prosthesis might be used for the treatment of a body vessel dilation such as an aneurysm, for example an abdominal aortic aneurysm. In this situation the prosthesis would once again comprise a radially expandable tubular member, having proximal and distal ends, an inner lumen and an outer surface, and a covering provided on the outer surface of that tubular member at least in the region of the proximal and distal ends. The covering may comprise a thrombogenic material as well as the agent that promotes swelling of the covering. As before, this agent is arranged to cause the covering to swell in size on exposure of the covering to contact with body fluid, for example blood. On delivery of this prosthesis into the vessel it is envisaged that the combined effect of the radial expansion of the tubular member and the swelling of the covering on the tubular member will cause the covering to expand outwardly into sealing contact with an inner wall of the vessel so as to reduce endoleak between the delivered and expanded prosthesis and the vessel.

The prostheses of both of the above examples of the present invention may also include one or more of the following attributes. For example, the tubular member, when expanded, may be generally cylindrical in shape, having an outwardly facing generally cylindrical surface on which the covering may be provided.

The covering may be arranged to conform to the contours of the wall of the vessel at the area of contact therebetween following delivery of the prosthesis. An effect of this would be to seal between the swollen covering and the vessel wall, and in the case of treatment of an AAA to reduce at least primary endoleak between the delivered prosthesis and the vessel.

In the hereinafter described and illustrated embodiments of endoluminal prosthesis in accordance with the present invention, the covering has an initial radial thickness prior to contact with the fluid and the swelling of the covering causes the covering to increase in radial thickness.

The agent may be arranged to swell due to hydrolysis.

The outer surface of the tubular member, for example a stent or graft, may be completely covered by the covering, or may simply be covered at the proximal and distal ends of the tubular member.

The thrombogenic material of the covering is, in the illustrated embodiments, arranged to promote haemostasis between the layers of the device itself located in the aneurysm, and is advantageously collagen or collagen sponge.

A preferred construction for the covering is to have the second layer comprise a thrombogenic material. The second layer advantageously comprises the outermost of the two layers.

Where the tubular member has a longitudinal central axis, relative to that axis the radial thickness of the covering may, for example, be between 0.5 and 1 mm. Furthermore, relative to that axis, where the covering has a radial thickness of *x* mm prior to swelling of the covering it has a radial thickness of at least 2*x* mm (preferably about 5*x*) following swelling of the covering.

The tubular member may need to be expanded, in use, by a balloon catheter or is, advantageously, self-expanding. In the latter case, the tubular member advantageously comprises a memory metal such as Nitinol.

The agent in the covering is arranged to cause the covering to swell on exposure of the covering on contact with fluid, for example blood, on delivery of the prosthesis into the vessel from a prosthesis delivery system.

In use, which is not part of the invention, the covering is arranged to expand outwardly into sealing contact with the inner surface of the vessel on both "sides" of the aneurysm so as to isolate the sac of the aneurysm from blood flowing along the vessel through the inner lumen of the expanded tubular member. It is preferred for the covering not to expand outwardly into sealing contact with the wall of the sac of the aneurysm, i.e. it is desirable for sealing contact to be established on either side of the sac of the aneurysm but not within the volume of the sac of the aneurysm itself

The prosthesis is thought to be particularly applicable for the treatment of aortic aneurysms, and for the prevention of primary endoleaks. Alternatively or additionally the prosthesis may be used for treating aneurysms elsewhere, for example in the iliac or femoral arteries.

According to a second aspect of the present invention, as appended in claim 12, there is provided the prosthesis of the above first aspect of the present invention in combination with a delivery instrument. This delivery instrument includes a sheath which is arranged substantially to cover the prosthesis as the prosthesis is being endolumenally delivered to a target site and substantially to shield the covering of the prosthesis from contact with blood. The sheath and the prosthesis are relatively movable so as to enable the prosthesis to be deployed from within the sheath at the target site to expose the covering of the prosthesis to contact with blood.

In a preferred arrangement the tubular member of the prosthesis is arranged to be mechanically expanded (either through the use of a separate tool such as a balloon catheter or through self-expansion) upon removal of the prosthesis from the sheath, and the agent is arranged to cause the covering to expand outwardly relative to the expanded tubular member. The expansion of both the tubular member and the covering combined, in use, to cause the exterior of the covering to be pressed into firm sealing contact with the inner lumen of the vessel at the target site, thereby contributing to reducing endoleak.

Where the inner lumen of the vessel at the target site is irregularly contoured, the exterior of the expanded covering is advantageously arranged to follow the irregular contours so as to effect the firm sealing contact with the inner lumen of the vessel.

According to a third aspect of the present invention there is provided a method of manufacturing an endoluminal prosthesis as appended in claim 14, comprising providing a substantially fluid impermeable and expandible tubular member having a proximal end, a distal end, an inner lumen and an outer surface and applying a covering to the outer surface of the tubular member. This covering may comprise both a thrombogenic material and an agent to promote swelling of the covering, the agent being arranged to cause the covering to swell outwardly on the tubular member towards the inner lumen of a vessel on delivery of the prosthesis into the vessel. The covering comprises first and second layers of material, with the first layer comprising the above mentioned agent and the second layer comprising a space-filling material.

The covering is applied to the tubular member by winding, around the tubular member, a first sheet comprising the agent and a second sheet containing the thrombogenic material. Advantageously, in the final prosthesis the second sheet forms the outermost layer of the covering.

The first and second sheets are simultaneously wound around the tubular member so as to produce a covering that comprises the two sheets interleaved together in a generally spiral arrangement.

According to an example which is not part of the invention, there is provided a method of treating a dilation in a blood vessel. The method may comprise the steps of:
implanting endovascularly the prosthesis of the present invention;
positioning the prosthesis at the site of the dilation to be treated; and
exposing the prosthesis to full contact with blood at said site, the full contact exposure of the agent to said blood causing the covering to swell in size to press the exterior of the swollen covering into sealing contact with the inner surface of the vessel.

In a preferred procedure the step of exposing the prosthesis to full contact with blood involves the removal of the prosthesis from a constraining sheath which sheath, when the prosthesis is constrained therein, substantially prevents contact between the blood and the covering.

Where the tubular member is self-expanding, upon the removal of the constraining sheath the tubular member self-expands in diameter and the covering independently expands in volume, the combined effect of these two expansions leading to the outer surface of the covering being pressed into firm sealing contact with the inner surface of the vessel at the site of the dilation, with the thrombogenic material of the covering being pressed into any surface irregularities in the inner surface of the vessel.

Advantageously, upon contact between the covering and the inner surface of a vessel at the site of the dilation, the thrombogenic material promotes clotting of the blood further to aid sealing between the prosthesis and the aneurysm.

Upon contact between the covering and the inner surface of the vessel at the site of an aneurysm, the covering is preferably only in contact with the inner surface of the vessel on either side of the aneurysm. In this way, some of the volume of the sac of the aneurysm is simply isolated rather than being closed, and any blood present in the sac of the aneurysm is cut off from blood flowing along the vessel through the inner lumen of the tubular member of the prosthesis.

As mentioned above, when positioned at the site of the aneurysm, the expanded prosthesis preferably bridges across the aneurysm so as to seal against the inner surface of the blood vessel on both sides of the aneurysm, but not within the aneurysm itself. Clotting of the blood within the aneurysm, associated with expansion of the prosthesis into firm sealing contact with the inner surface of the vessel, advantageously prevents primary endoleaks.

In use, clotting of blood at the proximal end of the tubular member promotes the ingrowth of cells and the incorporation of the proximal end of the tubular member into the vessel.

Embodiments of apparatus in accordance with the present invention will now be described, by way of example only, with reference to the accompanying drawings, in which:
Figure 1 is an axial cross-sectional view, across the line I-I in Figure 2, of a prior art graft positioned within the aorta of a young and/or healthy individual;
Figure 2 is a schematical longitudinal side elevation of the prior art graft of Figure 1 (to a reduced scale) shown positioned across an abdominal aortic aneurysm;
Figure 3 is a view similar to Figure 1, but shows a prior art graft positioned in an abnormal aneurysmal aorta of an unhealthy and/or elderly individual and is sectioned along the line III-III in Figure 4;
Figure 4 is a view similar to Figure 2, but showing the graft of Figure 3 (to a reduced scale) placed across an aortic aneurysm with both proximal and distal endoleak routes identified;
Figure 5 is a schematic drawing (not to scale) showing a prior art aortic aneurysm treatment device *in situ*;
Figure 6A shows, in side elevation, a tubular member of an endoluminal prosthesis which is not part of the invention
Figure 6B shows the tubular member of Figure 6A provided on its outer surface with a covering;
Figure 6C shows the prosthesis of Figure 6B with its covering hydrated and expanded;
Figure 6D shows a variant of the prosthesis of Figure 6B, in which the covering is provided only at the proximal end of the tubular member;
Figure 6E shows a second variant of the prosthesis of Figure 6B, in which the covering is provided at both the proximal and distal ends of the tubular member;
Figure 6F shows a third variant of the prosthesis of Figure 6B, in which the covering comprises a single layer or sheet of substantially homogenous construction;
Figure 6G shows a fourth variant of the prosthesis of Figure 6B, in which the covering comprises a multi-layered arrangement of the covering's layers;
Figures 7A - 7D illustrate a sequence of steps in an exemplary method of manufacturing a prosthesis of the present invention
Figure 8A shows, in schematic form, an initial stage in the placement of the prosthesis of Figure 6B, showing the prosthesis crimped down and prevented from radial expansion by a constraining sheath, whilst located in the aneurysm region of an aorta; and
Figure 8B shows a stage subsequent to that illustrated in Figure 8A, in which the constraining sheath has been removed and in which the tubular member has expanded, as has the covering provided on the tubular member, to force the covering strongly against the inner lumen of the aorta.

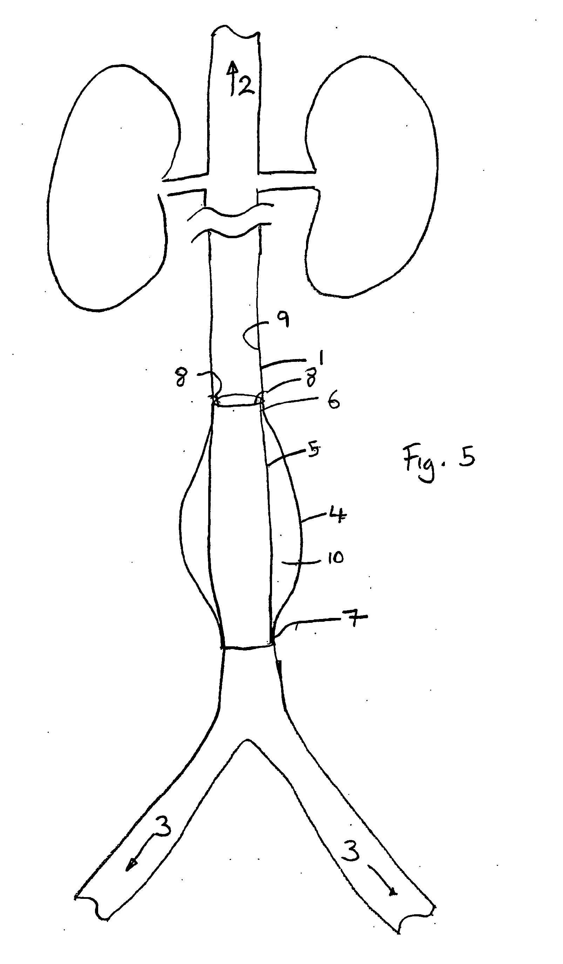

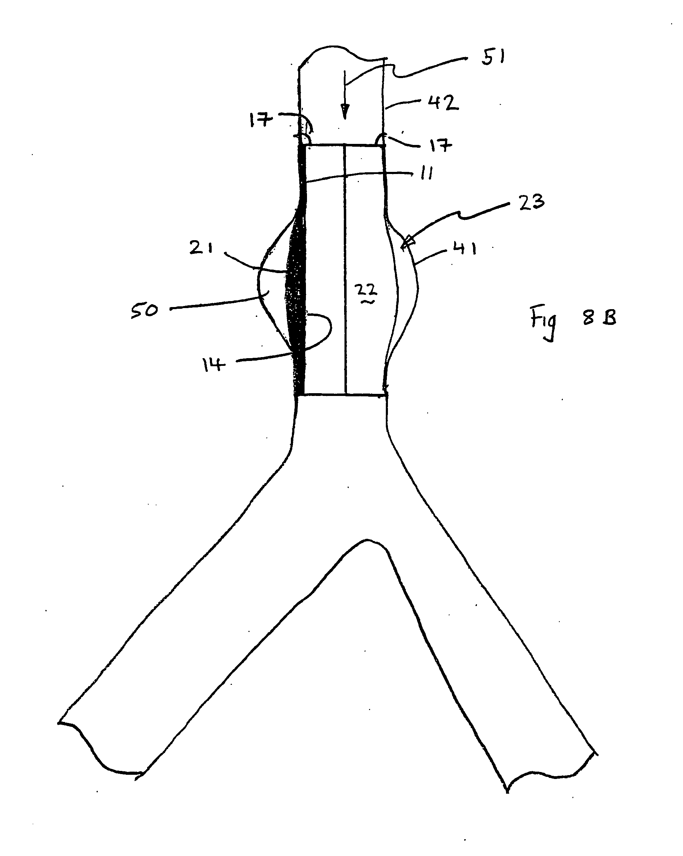

So as to make Figure 8B more meaningful in due course, Figure 5 is a schematic drawing (not to scale) of an aorta 1, with arrow 2 pointing in the direction of heart (not shown), against the flow of blood from the heart, and arrows 3 showing the direction of blood flow along the iliac vessels to the legs. The aorta 1 is shown having an aneurysm 4 formed therein. Positioned within the aneurysm 4 is a Dacron (Trade Mark)-covered prior art metal stent 5 provided at its proximal end 6 with hooks 8 engaging the inner lumen 9 of the aorta 1. As described in the above introduction to the specification, if the Dacron (Trade Mark)-covered metal stent 5 (which may or may not be self-expanding) does not fully engage the inner lumen 9 of the aorta 1 at the ends 6, 7 of the stent 5 (particularly at the proximal end 6 due to the direction of blood flow from the heart) leakage of blood into the sac 10 of the aneurysm 4 can occur, preventing clotting of blood in the sac and allowing the aneurysm to continue to grow in size, risking fatal rupture.

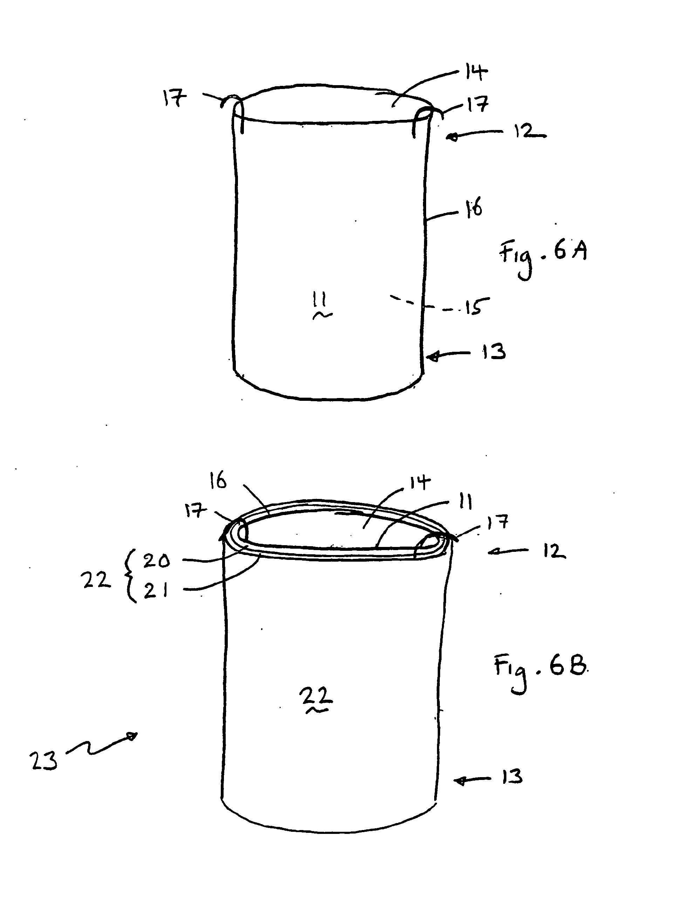

Figures 6A and 6B are two illustrations showing a first endoluminal prosthesis which is not part of the present invention being built up. The illustrations are shown as a series, and the components exaggerated in size, for reason of clarity.

Figure 6A is a tubular member 11 having a proximal end 12, a distal end 13, an inner lumen 14 and an outer surface 15. Provided at the proximal end 12 are a series of optional hooks 17 for engaging the inner lumen of a blood vessel, such as an aorta. The tubular member comprises a metal stent (not visible) in a fluid impermeable covering 16 as is conventional. The metal stent may advantageously be made ofNitinol so as to be self-expanding from a collapsed configuration upon delivery from a constraining sheath, but need not be self-expanding. As is also conventional, the covering 16 may comprise a sheet of Dacron (Trade Mark) or similar material fixed to the metal stent usually by a series of stitches (not shown). Although in the illustrated example the covering 16 is positioned externally of the metal stent, this need not be so - their positions could be reversed.

The construction of the tubular member 11 shown in Figure 6A is, thus far, conventional. The tubular member 11 is shown in its expanded configuration. As is conventional, in order to be delivered endolumenally, the member has to be capable of being collapsed down in order to reduce its overall diameter to one rendering it capable of endolumenal delivery.

Figure 6B shows the tubular member 11 of Figure 6A additionally provided on its outer surface with a covering 22 to form a prosthesis 23. In the illustrated example the covering 22 comprises a first layer or sheet 20 comprising an agent, and a second layer or sheet 21 comprising a thrombogenic material and/or space-filling material and not containing any of the agent. The first layer 20 extends once around the outer surface of the tubular member 11. The second layer 21 extends once around the first layer 20, so that the tubular member 11, the first layer 20 and the second layer 21 form three concentric rings.

The active ingredient in the agent might be one or more of the agents discussed below, but the preferred active agent is hyaluronic acid. Any thrombogenic material may comprise one or more of the materials discussed below. The preferred material for the second layer or sheet 21 is collagen sponge. The covering 22 provided on the outer surface of the expanded tubular 11 is shown in Figure 2B in its non-hydrated, or unexpanded or unswollen state.

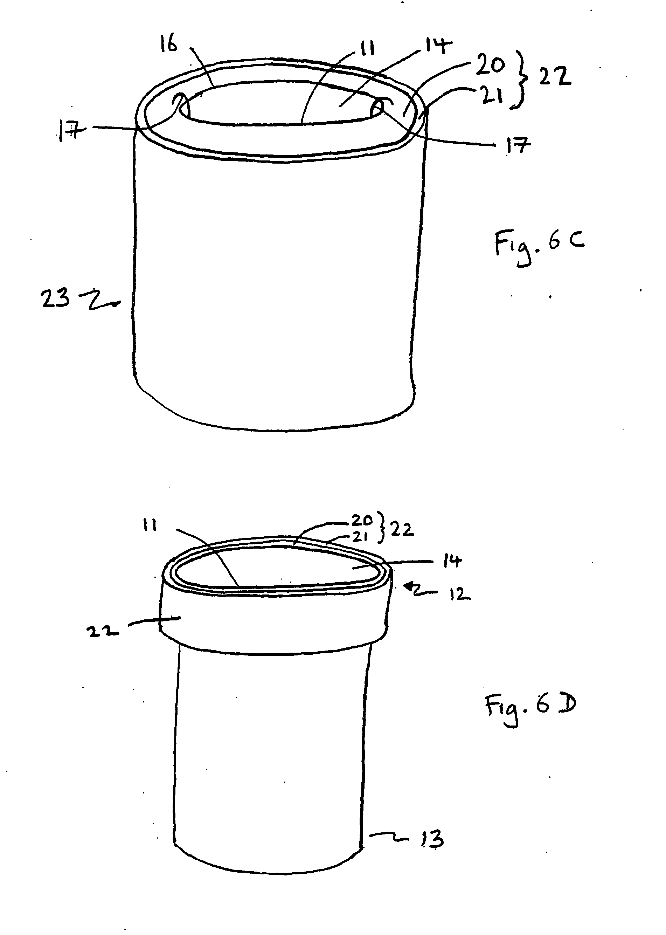

In contrast, Figure 6C shows the prosthesis 23 of Figure 6B in its hydrated state, such as it would be after exposure to blood. As can be seen, the hydration of the expansion-promoting agent has led to the first layer 20 and thus the covering 22 swelling and expanding in volume, thereby expanding radially outwardly. Space-filling material, such as collagen, in the second layer 21 will also hydrate too and may expand, but this is unlikely to be to the same extent as the expansion or swelling of the first layer 20.

As will be described below, by positioning the prosthesis 23 in a lumen and then hydrating the agent, the covering 22 can be caused to swell outwardly towards the inner lumen of the vessel so as to improve sealing contact between the exterior of the prosthesis 23 and the inner lumen of the vessel.

It should be appreciated that Figures 6A - 6C, for reasons of clarity, shows the prosthesis in a non-representative environment, in which it is not surrounded by a vessel. In normal use, the prosthesis would be positioned within the lumen of a vessel, prior to hydration of the agent, so as to provide a surface for the expansion in volume of the agent to increase the sealing contact with. In addition, Figure 6C exaggerates the amount of expansion of the covering 22, brought about by its hydration, for reasons of clarity.

Figure 6D shows a first variant in construction of the prosthesis 23 of Figures 6B and 6C. As can be seen, in Figure 6D the covering 22 is provided only at the proximal end 12 of the tubular member 11. In addition the hooks are omitted.

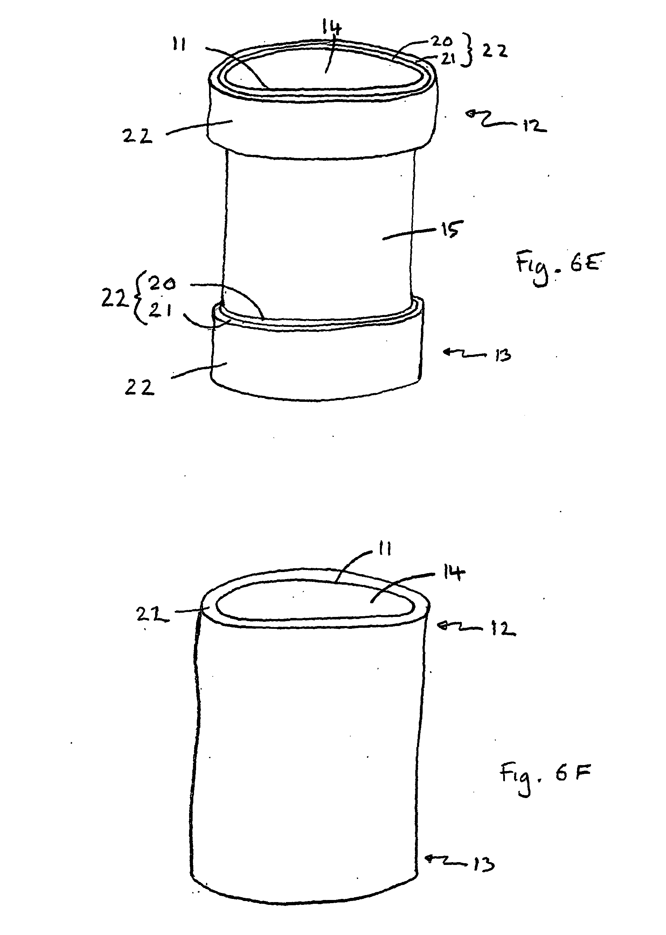

Figure 6E shows a second variant. In Figure 6E the covering 22 is present at both the proximal and distal ends 12,13 of the tubular member 11 but the central portion of the tubular member is not provided with the covering 22. When used as a graft for treatment of an AAA, the intention is that the coverings 22 at the ends should be aligned with the portions of the blood vessel on either side of the aneurysm sac, with the central portion of the tubular member being aligned with and bridging across the sac of the aneurysm.

Although in Figures 6B-6E the covering 22 is shown as being provided with a plurality of discrete layers or sheets 20,21, one comprising an agent to promote swelling and the other comprising a thrombogenic material, the covering 22 may comprise a single layer or sheet of substantially homogeneous construction, for example in which the thrombogenic material and the agent are combined together, as shown in the third variant of prosthesis in Figure 6F.

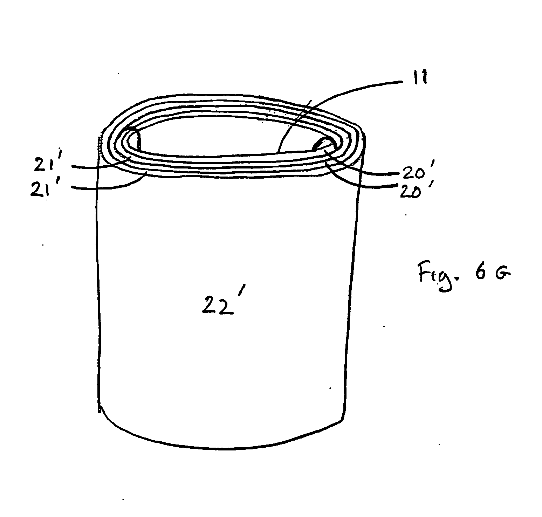

Equally, rather than simply comprising two concentrically arranged discrete layers or sheets 20, 21 as in Figures 6B-6E, the covering 22 might alternatively comprise a plurality of one or other (or both) types of layer or sheet. For example, as shown in the fourth variant in Figure 6G, the covering 22' comprises a pair of first layers or sheets 20' and a pair of second layers or sheets 21', arranged in the order: 20', 21' 20', 21', etc. The provision of a plurality of interleaved layers or sheets 21' is thought likely to enhance the thrombogenic effect and thus the sealing effect. When the prosthesis is deployed, and the plural layers in the covering are "wetted" in the blood flow, in addition to activating the agent in the first layers 20' blood may be trapped in the interstices between the adjacent layers 20', 21'. This blood trapped between the interleaved layers 20', 21' of the covering 22 will then clots, enhancing further the sealing ability of the prosthesis.

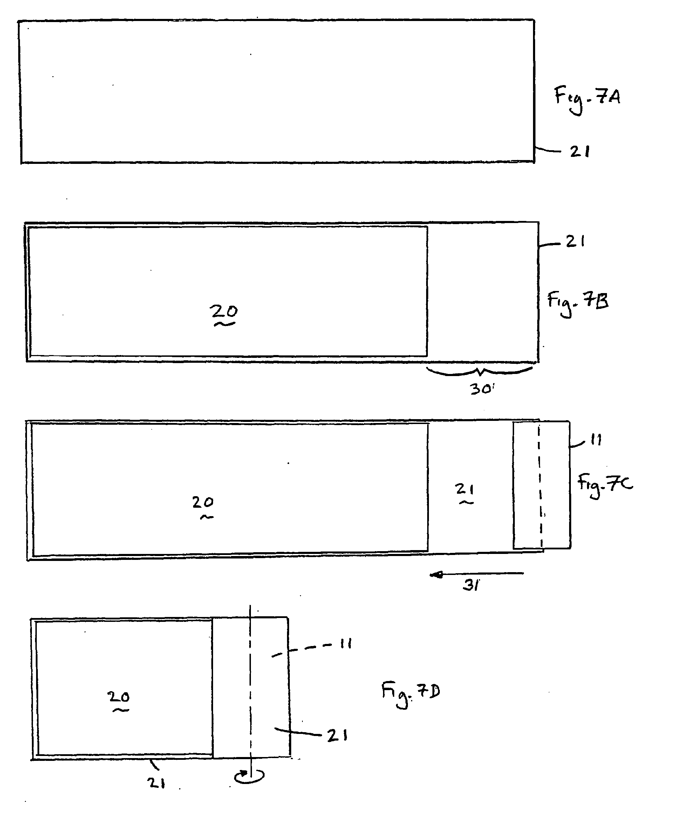

There will now be described, in conjunction with the sequence of Figures 7A-7D a sequence of steps in an exemplary method of manufacturing an endoluminal prosthesis 23 (in accordance with the invention). Where elements of the prosthesis 23 have a similar function to elements in embodiments or variants of prosthesis already described, these will be referenced with the same reference numerals.

As explained above in connection with Figure 6A, the underlying tubular member 11 of the endoluminal prosthesis 23 of the present invention can have a known construction and shape. Although, for reasons of simplicity, the endoluminal prosthesis shown in the drawings is a simple tubular stent or graft, the prosthesis could have any shape or configuration appropriate to its intended location in the body, for example, a Y-shaped trouser graft. The particular aspect of the prosthesis of the present invention is largely provided by the covering 22 on the outer surface of the tubular member 11.

Where the agent to promote swelling of the covering and the thrombogenic material are to be provided in separate sheets or layers, it is necessary first to manufacture the two different types of layer or sheet 20, 21.

The second layer or sheet 21 may comprise a rectangular sheet of collagen foam, as illustrated in Figure 7A. Alternatively, one can take a mixture of available collagen extract (usually presented in an acidic base), neutralise it and pour it into a mould in which it will set at room temperature. By regulating the depth of the liquid in the mould one can control the thickness of the resultant sheet. Once the material has set, it can be moved from the mould and have moisture removed from it by lightly compressing it, for example using blotting paper. The resultant sheet of material can either be a very large sheet, from which the sheet 21 illustrated in Figure 7A can be cut, or the mould may be appropriately sized.

To produce the first layer or sheet 20 one can taken hyaluronic acid in powdered form, mix it with distilled water, pour it into an appropriately shaped mould to a desired depth and freeze-dry it so as to form a flat sheet of material. This sheet 20, which can also either be cut to size or moulded in the appropriate size, can then be laid on the second sheet 21, as shown in Figure 7B. As long as there is no excess water in the second layer or sheet 21, the placing of the first sheet 20 on the second sheet 21 should not cause the hyaluronic acid to become hydrated.

In a preferred arrangement, the longitudinal element of the first sheet 20 is less than that of the second sheet 21. An uncovered tongue portion 30 of the second sheet 21 enables the tubular member 11, in its expanded form and containing a mandrel (not shown) to rigidify it, to be positioned on the right-hand edge (as drawn) of the tongue 30, in the manner shown in Figure 7C. By then rolling the tubular member 11 in the direction of arrow 31 in Figure 7C, the two sheets 20, 21 that form the covering 22 can be wrapped, in a generally spiral fashion, around the outer surface of the tubular member 11. Apart from the first layer of the covering 22 that will be exclusively comprised by the tongue portion 30 of the second sheet 21, the first and second sheets 20, 21 will be interleaved as they spiral around the central longitudinal axis of the tubular member 11. Figure 7D shows the arrangement part way through the wrapping process. If an uncovered tongue (not shown) of the second sheet 21 is also provided at the left hand end of the sheet, when the wrapping process is finished the end of that tongue may be stuck to the portion of the second sheet immediately below it to make the covering secure.

It will, of course, be appreciated that the covering 22 may be applied to the tubular member 11 in other ways, for example by spraying, dipping, coating etc. to form one or more layers.

In order to package the prosthesis ready for use, it is necessary to remove the mandrel (not shown) from the lumen of the tubular member 11 and to crimp down the prosthesis so as to reduce its outer diameter. The reduction in outer diameter might typically be from any one of 19.5, 21.5 or 23.5 mm to approximately 6 mm (for the prosthesis to be constrained in an 18 French inner diameter delivery system sheath) or from any one of 25.5,28,30.5,32 and 34 mm to 6.66 mm (to be constrained in an 20 French inner diameter delivery system sheath. The smaller the diametral dimension that can be achieved on crimping down the better, because smaller prosthesis may be more readily delivered and/or delivered via smaller vessels. The prosthesis would be crimped down onto a conventional delivery system in the manner of a conventional non-coated endoluminal prosthesis, and then vacuum packaged to keep it sterilized and dry. In its packaged state the crimped down prosthesis is preferably constrained radially by a constraining delivery system sheath. As will be described below, upon being released from this constraining sheath the prosthesis is able to be expanded radially by a balloon catheter (in the case of a non-self-expanding prosthesis) or will automatically self-expand (in the case of a self-expanding prosthesis).

It is anticipated that the prosthesis 23 will have a shelf-life of very many months (ideally years) if stored appropriately. When the time comes to use the prosthesis, for example to treat an aortic aneurysm endolumenally, the initial stages of the procedure will be as with a prior art uncoated prosthesis.

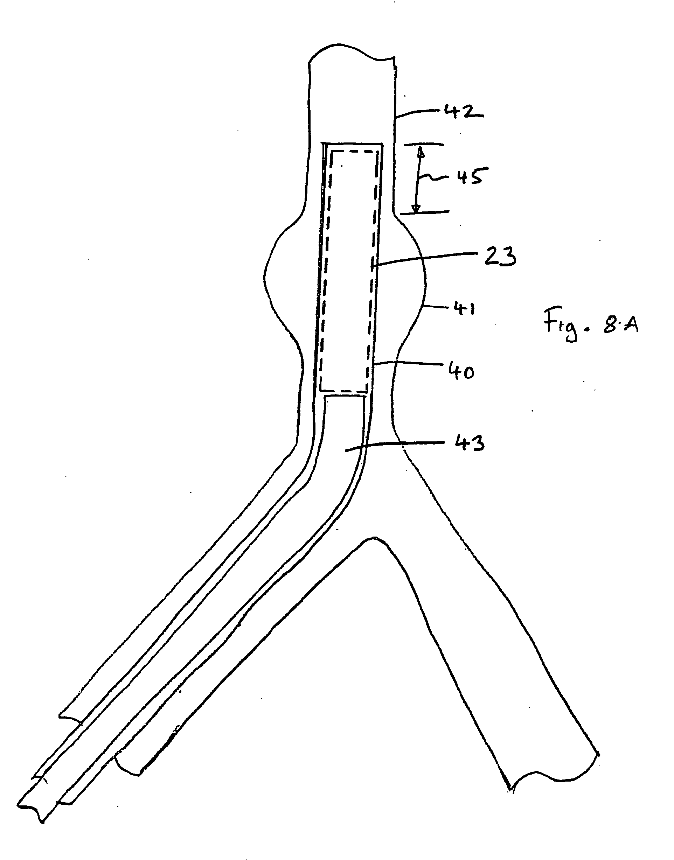

In a typical procedure, as shown in Figure 8A, the crimped down prosthesis 23, constrained against expansion by a constraining sheath 40, is fed endolumenally to the site of the aneurysm in a conventional manner. Using a fluoroscope the surgeon can position the prosthesis-containing portion of the sheath 40 bridging the aneurysm 41 to be treated. In Figure 8A the crimped down prosthesis 23, having the construction of the Figure 6B prosthesis, is shown in dotted lines.

As is conventional, it is anticipated that the prosthesis 23 will be supplied to the surgeon in a range of sizes, from which range to surgeon may pick the prosthesis with the most appropriate size. Typically the outer diameter of the unconstrained prosthesis 23 is chosen to be slightly oversize relative to the vessel (such as an aorta 42) into which the prosthesis is to be placed. For example, it is anticipated that a prosthesis 23 with an unconstrained outer diameter of 19.5 mm (measure across the covering 22 before swelling of the covering) would be suitable for use in a vessel with an native vessel inner diameter of 16.0 - 17.5 mm. In this way, the mechanical expansion of the prosthesis 23 (even before swelling of the covering 22) can be used to promote good sealing at the ends of the prosthesis 23, with subsequent swelling of the covering 22 further enhancing sealing. Exemplary sizes are listed below (in millimetres), with an unconstrained prosthesis outer diameter listed first, followed by an exemplary matching native vessel inner diameter in parentheses: 19.5 (16.0 - 17.5); 21.5 (17.5 - 19.5); 23.5 (19.5 - 21.5); 25.5 (21.0 - 23.5); 28.0 (23.0 - 25.5); 30.5 (25.0 - 27.5); 32.0 (26.5 - 29.5); 34.0 (28.5 - 31.5).

The procedure described below is for a self-expanding tubular member. In addition to constraining the crimped down prosthesis 23 against radial self-expansion, the sheath 40 has the effect of substantially shielding the prosthesis 23 from contact with blood in the aorta 42. Consequently, the agent that will promote swelling of the covering is substantially prevented from being hydrated prematurely.

Once the surgeon determines that the prosthesis 23 is appropriately positioned, by then holding the prosthesis 23 in place using a pusher 43 and withdrawing the sheath 40, the sheath 23 is left in position exposed to blood in the aorta 42. By continuing to hold the prosthesis 23 using the pusher 43 to resist the strong flow of blood in the aorta 42, in the case of a self-expanding prosthesis the prosthesis 23 will self-expand as shown in Figure 8B. As described above, by using a slightly oversize prosthesis 23 the mechanical expansion of the tubular member 11 alone may be sufficient as to bring the outside surface of the non-hydrated covering 22 into firm contact with the inner lumen of the aorta 42 - this is to be preferred because the hydration of the covering will then be able to push the covering hard against the wall of the aorta 42. Because the external profile of the tubular member 11 is general cylindrical, the mechanical expansion of the tubular member 11 alone may be unable to cope with small scale discontinuities or deviations, such as fissures or calcified plaques, in the inner lumen of the wall of the aorta which depart from a generally circular profile.

In Figure 8B the drawing of the prosthesis 23 is split down its centre for reasons of clarity. To the right-hand side of the centre-line of the prosthesis the expanded covering 22 is shown as being in contact with the inner lumen of the aorta at the ends of the prosthesis, but not in the centre of the prosthesis. The non-constant outside diameter of the expanded prosthesis is accounted for by differing degrees of expansion of the covering 22 along the length of the prosthesis. This is shown most readily in the left-hand half of Figure 8B. In the left-hand half the tubular member 11 is shown as being mechanically expanded, through its self-expansion mechanism, into contact with the narrowest portions of the inner lumen of the aorta. Where the aneurysm sac was previously, the outer layer or sheet 21 of the covering 22 is shown (in black) as having expanded part way into the volume of the aneurysm sac by virtue of the expansion of the first layer or sheet 20 of the covering 22.

In order to prevent primary endoleak it is sufficient for the covering 22 to be in contact with the inner lumen of the aorta at simply the end or ends of the prosthesis 23, as shown. It is not necessary for the full volume of the aneurysm sac to be taken up by the expanded covering 22, so that in the central portion of the sac the covering 22 is not required to expand to a size equal to the maximum dimension of the aneurysm sac. Instead, the central portion of the sac is intended to contain a volume of blood 50 that is cut off from blood 51 flowing along the aorta through the inner lumen 14 of the tubular member.

In addition to the mechanical expansion of the tubular member, the removal of the sheath 40 from the exterior of the prosthesis 23 means that the prosthesis is no longer substantially shielded from blood. The effect of blood contacting the covering 22 is to cause the active agent in the agent that promotes swelling of the covering to hydrate, causing the covering to swell expand in volume. The expansion in volume causes the external dimension of the prosthesis to increase beyond that which is achieved simply by mechanical expansion of the self-expanding tubular member 11. It is this swelling or expansion which causes the covering to be squeezed tightly against the inner lumen of the aorta 42 on either side of the aneurysm. Where the agent and thrombogenic material are provided in discrete layers or sheets 20,21, it is the thrombogenic material of the second layer or sheet 21 that is preferably outermost on the prosthesis. This thrombogenic material is squeezed into contact with the inner lumen of the aorta at the ends of the prosthesis by virtue of the combined (i) mechanical radial expansion of the tubular member 11 and (ii) the expansion of the covering 22 on the tubular member. It is this combined effect which causes the covering to expand outwardly into sealing contact with the inner surface of the aorta at the opposite ends of the aneurysm to reduce endoleak between the delivered and expanded prosthesis and the aorta.

It will be appreciated that the expansion of the covering 22 is more readily able to accommodate discontinuities within the inner lumen of the aorta than is mechanical expansion of the tubular member, for example discontinuities caused by atheroma.

The effect of the expansion of the covering 22 is to improve sealing between the prosthesis and the inner lumen of the aorta, particularly in cases where the inner lumen is irregular in shape from plaque and/or fissured.

The agent that promotes swelling of the covering is thought likely to cause the covering to expand outwardly in fewer than 5 minutes, preferably fewer than a couple of minutes. The timing can be modified through the use of differing amounts of the active agent, making it possible to fabricate 'fast' and 'slow' expanding versions of the prosthesis. The expansion should advantageously not, however, be so quick as to prevent the surgeon from repositioning the prosthesis if, on withdrawal of the constraining sheath 40, the prosthesis is moved out of position.

In use, it is envisaged that the prosthesis should extend by a distance (reference 45) approximately 1 - 3cm beyond each end of the aneurysm, both proximally, at the 'neck' of the aneurysm, and distally, most likely into one or other of the iliac arteries.

Once the tubular member has mechanically expanded and the covering has also expanded, the delivery apparatus may be withdrawn. In the case where the prosthesis is provided with hooks the combined effect of those hooks and the expansion of the prosthesis against the inner wall of the aorta should be sufficient as to keep the prosthesis in place, despite the strong blood flow.

It is envisaged that the prosthesis will be provided to a surgeon in a range of different sizes, for example differing in outside diameter by 2mm steps across the range, and different swelling rates. The surgeon will thus be able to select a prosthesis of an appropriate size and deployment speed. In its non-hydrated state, the covering 22 may have a radial thickness of, for example, 0.5mm, but when hydrated may have a radial thickness of approximately 2 - 3mm (or more). Consequently, providing a 0.5mm thick covering around a tubular member, where the tubular member has an outside diameter of 30mm in its mechanically self-expanded state, will cause the non-hydrated prosthesis to have an outside diameter of approximately 31mm. Upon hydration of the covering, however, the outside diameter of the self-expanded prosthesis will be approximately 34 - 36mm, i.e. an increase in diameter of approximately 35mm.

Although in the embodiments described to date the tubular member 11 has generally been cylindrical, it may take other shapes which are not part of the invention as claimed, for example having at least.one divergent arm (or even two, to have an inverted-Y shape) so as to enable the prosthesis to be sited at the distal branching of the aorta into the iliac arteries.

Once the prosthesis has been deployed and the agent has caused the covering to swell and expand outwardly to press the thrombogenic material against the inner lumen of the aorta (and into any surface irregularities in the inner lumen of the aorta) on either side of the aneurysm, the active ingredient in the thrombogenic material, for example collagen, activates the 'clotting cascade'. Clotting of blood external to the tubular member of the prosthesis further promotes good sealing between the prosthesis and the inner lumen of the aorta, reducing primary endoleaks. Generally speaking, after a few weeks, the risk of endoleaks lessens, because the cells of the inner lumen of the aorta grow on to the inner lumen of the tubular member of the prosthesis yet further, assisting sealing and location of the prosthesis. It is anticipated that, once deployed, blood will over time occupy some of the volume that was occupied by the expanded expansion-promoting agent, but this blood should clot very quickly due to the proximity to thrombogenic material. Clotting at the surface of the thrombogenic material (e.g. collagen) is likely also to be promoted by localised dehydration of the of the blood layer by the swelling material with which it is in direct contact. Hence blood proteins, coagulation factors and platelets for example should all be concentrated onto and 'sucked' onto the thrombogenic material. Any remaining swelling-promoting agent may, over time, turn to gel and pass down harmlessly into the sac of the aneurysm.

### Preferred Agent to Promote Swelling

Although the agent to promote swelling is disclosed in the preferred embodiment as comprising hyaluronic acid originating in powdered form, the agent can be any significantly or substantially charged polymer molecule or even monomer which is, or can be, aggregated to form very high molecular weight complexes. Advantageously, the agent must be (or be able to rapidly become, for example within a few seconds or at least minutes) very large in molecular dimensions, ie. >>1 million molecular weight. Ideally, it should be or be able to rapidly become large enough to be trapped within or excluded by the pores in the thrombogenic material (commonly this may be in the range 100nm to 100µm in any one dimension). Trapping or exclusion might be through total aggregate dimensions, very high aspect ratio (fibres with high length to width) or by having substantial physico-chemical affinity for the second, trapping element (eg ligand-receptor activity, complementary chemical binding sites etc.).

Ideally the agent that promotes swelling is a very large soluble or insoluble polymer solid. If required, aggregation of a monomeric swelling element (eg {single, self aggregating, compound or combination of interacting molecules} drug, small molecule, protein, polysaccharide, synthetic polymer-precursor) could be envisaged, providing its aggregation was complete very rapidly after insertion and exposure to blood or its components.

Rapid swelling of the first element (ie generation of the molecular motor function) is ideally by uptake of fluid from surroundings by osmotic flow, out of the surrounding fluids and tissues to hydrate the swelling element. This is best driven by a high and controllable fixed charge density on the polymer or aggregate. Commonly and most simply this is provided by a number of naturally occurring (or, in time, synthetic) charged polysaccharides, ideally hyaluronan but also chondroitin sulphates, dermatan and its sulphates and keratin and its sulphates, all well known in the field. Other neutral polysaccharides could be used, particularly if they were modified to carry high fixed charge densities. The fixed charge density must be sufficient to cause the polymer to swell rapidly on first exposure to aqueous fluids, and sufficient to be able to draw this fluid from surrounding biological tissues against their own hydration gradients. Ideally, this means that the swelling polymer will rapidly form an expanding gel immediately on exposure to aqueous fluids, at the same time generating significant mechanical force associated with that swelling, again ideally focussed on tissue or implant layers adjacent to the swelling element (ie localised forces, especially between layers). It is chemically possible to modify almost any polymer to introduce such high fixed charges, though clearly many will be or will generate undesired (toxic) effects in the body. Other candidate might be clinically acceptable polymers (ideally biodegradable), proteins (ideally self or catalytically/enzyme aggregating, eg amphyphillic peptides, extracellular matrix proteins, fibrin(ogen)), polynucleic acids, even (though less likely because of their dominant hydrophobic nature) lipids. Ideally, swelling needs to be controllable in terms of rate and even force generated and this would require that the fixed charge density be controlled and even constant over the average volume of the swelling element, otherwise swelling and occlusion in any given time frame or against any given flow pressure could not be guaranteed or predicted.

### Thrombogenic material

Although in the illustrated embodiment the thrombogenic material includes collagen foam, other materials may be used to provide a haemostatic effect. Ideally, the thrombogenic material would be a clinically acceptable solid polymeric material with controllable pores (∼ 100 nm to 100µm average range diameter though potentially larger or smaller than this). It should have sufficient material strength and integrity to retain its shape and structure within its layer position, against competing forces, fluid shears and especially compression. It must have a lower inherent swelling pressure (fixed charge density) than the swelling element. Fibrous extracellular matrix & structural proteins (collagen, clotting proteins (fibrin, fibronectin) silks are ideal for this, but synthetic polymers, neutral polysaccharides (chitin, cellulose, starch) could be used to some extent, particularly after chemical modifications, as long as they could fulfil the mechanical requirements. Fibrous or porous solid polymers (random or non-random) are best, others include granular materials (large particles) less desirable are small particle granules, emulsions, micro-beads etc. if used without restraining/enclosing layers.

The idea has two parallel mechanisms, (i) physical occlusion and (ii) promotion of haemostasis. Some trapping elements could act using only one of these, though less effectively and less rapidly. Aggregated, solid, native collagen is ideal as it is a naturally strong, flexible material, low swelling potential, clinically acceptable (low antigenicity etc) yet it promotes rapid platelet aggregation and degranulation, i.e. initiates the full clotting cascade. Other versions might use immobilised factors, peptide sequences or enzymes to mimic this activity, e.g. by initiating platelet aggregation/degranulation and or fibrin aggregation from fibrinogen (e.g. most simply by provision of active thrombin). Such mechanisms might also be used with collagen to further enhance, speed up its role where needed. Significant non-ideal, other candidates for this might be chemically modified synthetic polymers, aggregated non-structural (globular) proteins such albumin, immunoglobulins, neutral polysaccharides such as cellulose, chitin etc), even inorganic backbone materials, if sufficiently flexible, such as glass (eg soluble phosphate) fibres, ceramic particles, kaolin, etc. However, many of these are not mechanically suitable and or would cause significant clinical problems if/when they were released into the rest of the body, eg generating tissue toxicity, immune reactions/inflammation, dangerous coagulations and small vessel constrictions.

Longer term stabilisation of the interface between the prosthesis and the vessel with which it is in contact might be accelerated by the addition of factors such as TGF-β isoforms, for example to the thrombogenic material, to promote rapid fibrous ingrowth into the newly formed clot.

### Treatment of the Non-Anemysmal Dilations (not part of the invention)

The description and drawings thus far primarily concern the treatment of aneurysmal dilation and the prevention of primary endoleak. The prosthesis of the present invention does, however, find uses in the treatment of non-aneurysmal dilations where the benefits do not relate to primary endoleak prevention.

A further use envisaged for the endoluminal prosthesis of the present invention concerns a balloon angioplasty procedure. In such a procedure, it is common to use a balloon catheter to dilate a narrowing in an artery and then to insert a stent into the dilated artery so as to assist in keeping the artery open. It is also know for such stents to be a drug or medicament eluting stent, which elute the drug or medicament over time. Rather than using a prior art stent following a balloon angioplasty procedure it is envisaged that the endoluminal prosthesis of the present invention could be used instead. In such a situation, it is unlikely that the covering on the tubular member of the prosthesis would comprise thrombogenic material, but it might if the underlying body of the stent was provided with a fluid impermeable membrane so as to minimise entry of the thrombogenic material into blood passing through the lumen of the tubular member. Instead, it is envisaged that the covering, preferably the second layer of material in the covering, would comprise a space-filling material such as collagen which is loaded with drug or medicament. As in the earlier embodiments, the expansion of the tubular member and the swelling of the covering provided on the outer surface of the tubular member would have the effect of forcing the exterior of the swollen covering into intimate contact with the surrounding inner wall of the vessel at the site of the balloon angioplasty. The sponge-like nature of the covering, together with the intimate contact between the covering and the surrounding inner wall of the vessel, could be used to promote "incorporation" of the prosthesis into the wall of the vessel. Furthermore, if the covering was provided with a drug or medicament, the intimate contact between the drug or medicament-loaded covering and the surrounding inner wall of the vessel could be used to promote healing between the stent and the vessel wall.

In other words, the dilation being treated can be as a result of the surgical expansion of an unwanted constriction, rather than a naturally occurring unwanted dilation such as an aneurysm.

### Potential Attributes

Attributes foreseen for the endoluminal prosthesis of the present invention, whether used for the treatment of non-aneurysmal dilations or not, may include one or more of the following.
1. To effectively close spaces between the prosthesis and the surrounding inner wall of the vessel.
2. To enable outward expansion of the covering away from the tubular member, to varying degrees.
3. To enable expansion only once the prosthesis has been delivered from a surrounding sheath.
4. To be able to be crimped for containment in a delivery sheath.
5. To have a reasonable shelf life.
6. To be easy to manufacture and to readily reproducible.
7. To be cost efficient.
8. To be non-toxic and avoid causing significant complications.
9. To promote further "incorporation" and healing between the prosthesis and the surrounding vessel walls.
10. To be applicable to any size of graft or stent available now or in the future.
11. To be able to have a desiccated, crimped diameter of approximately 0.5mm.
12. To expand in diameter, on swelling, to greater than 3mm
13. To swell in less than two minutes.
14. To promote thrombis formation between the layers of the covering.

## Claims

1. An endoluminal prosthesis (23) for use in the treatment of a dilation in a surrounding body vessel, the prosthesis comprising:
a radially expandible tubular member (11) having a proximal end, a distal end, an inner lumen, an outer surface and a circumference; and
a covering (22) provided on the outer surface of the tubular member (11) at least in the region of the proximal and distal ends, the covering comprising a first (20) layer of material, the first layer (20) comprising an agent that promotes swelling of the covering on exposure of the covering to contact with body fluid;
wherein the tubular member (11) and the covering (22) are constructed and arranged so that, on delivery of the prosthesis (23) into the surrounding body vessel, the tubular member (11) will expand radially and the covering (22) will swell in volume on the tubular member (11), said expansion and swelling forcing the exterior of the swollen covering (22) to be pressed into any surface irregularities in the inner wall of the surrounding body vessel so as to make intimate sealing contact with the inner wall, **characterized in that** : the covering (22) comprises a second layer (21) of material which comprises a space-filling material, the first (20) and second (21) layers being interleaved and extending generally spirally around the outer surface of the tubular member (11).

2. A prosthesis as claimed in claim 1, wherein the space-filling material is generally sponge-like.

3. A prosthesis as claimed in claim 1 or claim 2, wherein the space-filling material comprises collagen or the like.

4. A prosthesis as claimed in any one of the preceding claims, wherein the space-filling material is loaded with a drug or medicament, which drug or medicament may optionally be arranged to elute from the prosthesis following delivery of the prosthesis.

5. A prosthesis as claimed in any one of the preceding claims, wherein:
the covering (22) extends along substantially the complete length of the outer surface of the tubular member (11), and/or
the radially expandable tubular member (11) is, when expanded, generally cylindrical in shape, having an outwardly facing generally cylindrical surface, and the covering (22) is provided on that outwardly facing surface.

6. A prosthesis as claimed in any one of the preceding claims, wherein the covering (22) is arranged to conform to the contours of the wall of the vessel at the area of contact therebetween following delivery of the prosthesis so as to seal between the swollen covering (22) and the vessel wall and to reduce at least primary endoleak between the delivered prosthesis and the vessel.

7. A prosthesis as claimed in any one of the preceding claims, wherein the agent is arranged to swell due to hydrolysis and optionally includes an active agent and that active agent comprises one or more of the following:
hyaluronan, chondroitin sulphates, dermatan and its sulphates, keratin and its sulphates, and heparin, and yet further optionally the agent is hyaluronic acid.

8. A prosthesis as claimed in any one of the preceding claims, wherein the covering (22) includes thrombogenic material to promote haemostasis in the dilation, which thrombogenic material optionally is present only in the second layer (21) of the covering (22) and optionally comprises one or more of the following:
collagen, clotting proteins, fibrin, fibronectin, silks, synthetic polymers, neutral polysaccharides, chitin, cellulose, starch.

9. A prosthesis as claimed in any one of the preceding claims, wherein the tubular member (11) has a longitudinal central axis and, relative to that axis, the covering has a radial thickness of x mm prior to swelling of the covering (22) and a radial thickness of at least 2*x* mm following swelling of the covering.

10. A prosthesis as claimed in any one of the preceding claims, wherein the tubular member (11) comprises a stent or graft.

11. A prosthesis as claimed in any one of the preceding claims, wherein:
the agent is arranged to cause the covering to swell on exposure of the covering (22) to contact with body fluid on delivery of the prosthesis (23) into the vessel from a prosthesis delivery system; and/or
when the dilation is an aneurysm, the covering (22) is arranged to expand outwardly into sealing contact with the inner surface of the vessel around the aneurysm so as to isolate the sac of the aneurysm from blood flow along the vessel through the inner lumen of the expanded tubular member (11), and/or
the prosthesis (23) is an aortic aneurysm treatment device.

12. A prosthesis delivery system, comprising the prosthesis (23) of any one of the preceding claims and a delivery instrument, wherein the delivery instrument includes a sheath (40) which is arranged substantially to cover the prosthesis (23) as the prosthesis (23) is being endolumenally delivered to a target site and substantially to shield the covering (22) of the prosthesis (23) from contact with body fluid, the sheath (40) and the prosthesis (23) being relatively movable so as to enable the prosthesis (23) to be deployed from within the sheath (40) at the target site to expose the covering of the prosthesis (23) to contact with body fluid.

13. A system as claimed in claim 12, wherein the inner wall of the vessel at the target site is irregularly contoured and the exterior of the expanded covering (22) is arranged to follow the irregular contours to effect intimate sealing contact with the inner wall of the vessel.

14. A method of manufacturing an endoluminal prosthesis, comprising:
providing a substantially fluid impermeable and expandible tubular member (11) having a proximal end, a distal end, an inner lumen, an outer surface and a circumference; and
applying a covering (22), comprising first (20) and second (21) layers of material, to the outer surface of the tubular member (11) simultaneously winding first (20) and second sheets (21) of material around the tubular member (11) to form the first (20) and second (21) layers respectively and to produce a covering (22) that comprises the two sheets interleaved together in a generally spiral arrangement;
wherein the first layer (20) comprises an agent to promote swelling of the covering so as to cause the covering to swell outwardly on the tubular member (11) on delivery of the prosthesis into a cause the covering to swell outwardly on the tubular member (11) on delivery of the prosthesis into a surrounding body vessel and the second layer (21) comprising a space-filling material, wherein the tubular member (11) and covering (22) are constructed and arranged so that, on delivery of the prosthesis (23) into the surrounding body vessel, the tubular member (11) will expand radially and the covering (22) will swell in volume on the tubular member (11) so as to press the exterior of the swollen covering (22) into any surface irregularities in the inner wall of the surrounding body vessel so as to make intimate sealing contact with the inner wall.

15. A method as claimed in claim 14 wherein the manufactured prosthesis (23) is as claimed in any one of claims 1 to 11.

## Patentansprüche

1. Endoluminale Prothese (23) zur Verwendung bei der Behandlung einer Dilatation in einem umgebenden Körpergefäß, wobei die Prothese umfasst:
ein radial weitbares röhrenförmiges Element (11) mit einem proximalen Ende, einem distalen Ende, einem inneren Lumen, einer Außenfläche und einem Umfang; und
eine Abdeckung (22), die an der Außenfläche des röhrenförmigen Elements (11) zumindest in dem Bereich des proximalen und des distalen Endes vorgesehen ist, wobei die Abdeckung eine erste (20) Schicht eines Materials umfasst, wobei die erste Schicht (20) ein Mittel umfasst, das ein Anschwellen der Abdeckung bei Inkontaktbringen der Abdeckung mit Körperfluid fördert;
wobei das röhrenförmige Element (11) und die Abdeckung (22) so konstruiert und ausgelegt sind, dass bei Zufuhr der Prothese (23) in das umgebende Körpergefäß sich das röhrenförmige Element (11) radial weitet und die Abdeckung (22) vom Volumen her an dem röhrenförmigen Element (11) anschwillt, wobei das Weiten und das Anschwellen ein Pressen der Außenseite der angeschwollenen Abdeckung (22) in etwaige Oberflächenunregelmäßigkeiten in der Innenwand des umgebenden Körpergefäßes erzwingen, um engen dichtenden Kontakt mit der Innenwand herzustellen, **dadurch gekennzeichnet, dass:**
die Abdeckung eine zweite Schicht (21) eines Materials umfasst, das ein raumfüllendes Material umfasst, wobei die erste (20) und die zweite (21) Schicht überlappend sind und sich im Allgemeinen spiralförmig um die Außenfläche des röhrenförmigen Elements (11) erstrecken.

2. Prothese nach Anspruch 1, **dadurch gekennzeichnet, dass** das raumfüllende Material im Allgemeinen schwammartig ist.

3. Prothese nach Anspruch 1 oder 2, **dadurch gekennzeichnet, dass** das raumfüllende Material Collagen oder dergleichen umfasst.

4. Prothese nach einem der vorhergehenden Ansprüche, **dadurch gekennzeichnet, dass** das raumfüllende Material mit einem Arzneimittel oder einem Medikament beladen ist, wobei das Arzneimittel oder Medikament optional so ausgelegt sein kann, dass es nach Zufuhr der Prothese aus der Prothese eluiert.

5. Prothese nach einem der vorhergehenden Ansprüche, **dadurch gekennzeichnet, dass**:
sich die Abdeckung (22) entlang im Wesentlichen der gesamten Länge der Außenfläche des röhrenförmigen Elements (11) erstreckt und/oder
das radial weitbare röhrenförmige Element (11) im geweiteten Zustand im Allgemeinen von zylindrischer Form ist, wobei es eine nach außen weisende, im Allgemeinen zylindrische Oberfläche aufweist, und die Abdeckung (22) auf dieser nach außen weisenden Oberfläche vorgesehen ist.

6. Prothese nach einem der vorhergehenden Ansprüche, **dadurch gekennzeichnet, dass** die Abdeckung (23) so ausgelegt ist, dass sie nach Zufuhr der Prothese den Konturen der Wand des Gefäßes in dem Kontaktbereich dazwischen entspricht, um zwischen der angeschwollenen Abdeckung (22) und der Gefäßwand abzudichten und zumindest primäres Endolecken zwischen der zugeführten Prothese und dem Gefäß zu verringern.

7. Prothese nach einem der vorhergehenden Ansprüche, **dadurch gekennzeichnet, dass** das Mittel so ausgelegt ist, dass es aufgrund von Hydrolyse anschwillt, und optional einen Wirkstoff umfasst und dass der Wirkstoff ein oder mehrere der Folgenden umfasst:
Hyaluronan, Chondroitinsulfate, Dermatan und seine Sulfate, Keratin und seine Sulfate und Heparin und dass des Weiteren das Mittel optional Hyaluronsäure ist.

8. Prothese nach einem der vorhergehenden Ansprüche, **dadurch gekennzeichnet, dass** die Abdeckung (22) thrombogenes Material umfasst, um Hämostase bei der Dilatation zu fördern, wobei das thrombogene Material optional nur in der zweiten Schicht (21) der Abdeckung (22) vorhanden ist und optional ein oder mehrere der Folgenden umfasst:
Collagen, Gerinnungsproteine, Fibrin, Fibronektin, Seiden, synthetische Polymere, neutrale Polysaccharide, Chitin, Cellulose, Stärke.

9. Prothese nach einem der vorhergehenden Ansprüche, **dadurch gekennzeichnet, dass** das röhrenförmige Element (11) eine Längsmittelachse aufweist und die Abdeckung im Verhältnis zu dieser Achse eine radiale Dicke von x mm vor dem Anschwellen der Abdeckung (22) und eine radiale Dicke von mindestens 2x mm nach dem Anschwellen der Abdeckung aufweist.

10. Prothese nach einem der vorhergehenden Ansprüche, **dadurch gekennzeichnet, dass** das röhrenförmige Element (11) einen Stent oder ein Transplantat umfasst.

11. Prothese nach einem der vorhergehenden Ansprüche, **dadurch gekennzeichnet, dass:**
das Mittel so ausgelegt ist, es bei Zufuhr der Prothese (23) in das Gefäß von einem Prothesenzufuhrsystem ein Anschwellen der Abdeckung bei Inkontaktbringen der Abdeckung (22) mit Körperfluid hervorruft; und/oder
wenn die Dilatation ein Aneurysma ist, die Abdeckung (22) so ausgelegt ist, dass sie sich nach außen in dichtenden Kontakt mit der Innenfläche des Gefäßes um das Aneurysma weitet, um den Aneurysmasack von Blutfluss entlang des Gefäßes durch das innere Lumen des geweiteten röhrenförmigen Elements (11) zu isolieren; und/oder
die Prothese (22) eine Vorrichtung zur Behandlung eines Aortenaneurysmas ist.

12. Prothesenzufuhrsystem, welches die Prothese (23) nach einem der vorhergehenden Ansprüche und ein Zufuhrinstrument umfasst, **dadurch gekennzeichnet, dass** das Zufuhrinstrument eine Hülse (40) umfasst, die so ausgelegt ist, dass sie im Wesentlichen die Prothese (23) bedeckt, wenn die Prothese (23) endoluminal zu einem Zielort befördert wird, und dass sie im Wesentlichen die Abdeckung (22) der Prothese (23) vor Kontakt mit Körperfluid abschirmt, wobei die Hülse (40) und diese Prothese (23) verhältnismäßig beweglich sind, um der Prothese (23) ein Befördern aus der Hülse 40) heraus an den Zielort zu ermöglichen, um die Abdeckung der Prothese (23) mit dem Körperfluid in Kontakt zu bringen.

13. System Anspruch 12, **dadurch gekennzeichnet, dass** die Innenwand des Gefäßes an der Zielstelle unregelmäßig konturiert ist und die Außenseite der geweiteten Abdeckung (22) so ausgelegt ist, dass sie den unregelmäßigen Konturen folgt, um einen engen abdichtenden Kontakt mit der Innenwand des Gefäßes zu bewirken.

14. Verfahren zum Herstellen einer endoluminalen Prothese, welches umfasst:
Vorsehen eines im Wesentlichen fluidundurchlässigen und weitbaren röhrenförmigen Elements (11) mit einem proximalen Ende, einem distalen Ende, einem inneren Lumen, einer Außenfläche und einem Umfang; und
Anbringen einer Abdeckung (22), die eine erste (20) und zweite (21) Schicht eines Materials umfasst, an der Außenfläche des röhrenförmigen Elements (11) durch gleichzeitiges Wickeln der ersten (20) und zweiten Lage (21) von Material um das röhrenförmige Element (11), um die erste (20) bzw. zweite (21) Schicht zu bilden und eine Abdeckung (22) zu erzeugen, die die beiden Lagen einander überlappend in einer im Allgemeinen spiralförmigen Anordnung umfasst;
wobei die erste Schicht (20) ein Mittel umfasst, um ein Anschwellen der Abdeckung zu fördern, um bei Zufuhr der Prothese in ein umgebendes Körpergefäß ein Anschwellen der Abdeckung an dem röhrenförmigen Element (11) nach außen zu bewirken, und die zweite Schicht (21) ein raumfüllendes Material umfasst, wobei das röhrenförmige Element (11) und die Abdeckung (22) so konstruiert und ausgelegt sind, dass sich bei Zufuhr der Prothese (23) in das umgebende Körpergefäß das röhrenförmige Element (11) radial weitet und die Abdeckung (22) vom Volumen her an dem röhrenförmigen Körper (11) anschwillt, um die Außenseite der angeschwollenen Abdeckung (22) in etwaige Obertlächenunregelmäßigkeiten in der Innenwand des umgebenden Körpergefäßes zu drücken, um engen abdichtenden Kontakt mit der Innenwand herzustellen.

15. Verfahren nach Anspruch 14, **dadurch gekennzeichnet, dass** die hergestellte Prothese (23) gemäß einem der Ansprüche 1 bis 11 ist.

## Revendications

1. Prothèse endoluminale (23) à utiliser dans le traitement d'une dilatation d'un vaisseau corporel situé autour, la prothèse comprenant :
un élément tubulaire (11), expansible radialement, ayant une extrémité proximale, une extrémité distale, un conduit intérieur, une surface extérieur et une circonférence ; et
une enveloppe (22) placée sur la surface extérieure de l'élément tubulaire (11) au moins dans la région des extrémités proximale et distale, l'enveloppe comprenant une première couche (20) de matériau, la première couche (20) comprenant un agent qui stimule le gonflement de l'enveloppe lorsque l'enveloppe est exposée au contact d'un fluide corporel ;
dans laquelle l'élément tubulaire (11) et l'enveloppe (22) sont conçus et disposés de telle sorte que, lors de la mise en place de la prothèse (23) dans le vaisseau corporel situé autour, l'élément tubulaire (11) se déploie radialement et l'enveloppe (22) gonfle en augmentant de volume sur l'élément tubulaire (11), lesdits déploiement et gonflement forçant l'extérieur de l'enveloppe gonflée (23) à être pressée dans toutes les irrégularités de surface de la paroi intérieure du vaisseau corporel situé autour de manière à former un bon contact d'étanchéité avec la paroi intérieure, **caractérisée en ce que** : l'enveloppe (22) comprend une deuxième couche (21) de matériau qui comprend un matériau de remplissage d'espace, les première (20) et deuxième (21) couches étant intercalées et s'étendant généralement en spirale autour de la surface extérieure de l'élément tubulaire (11).

2. Prothèse selon la revendication 1, dans laquelle le matériau de remplissage d'espace est généralement de type spongieux.

3. Prothèse selon la revendication 1 ou la revendication 2, dans laquelle le matériau de remplissage d'espace comprend du collagène ou équivalent.

4. Prothèse selon l'une quelconque des précédentes revendications, dans laquelle le matériau de remplissage d'espace est chargé d'un produit thérapeutique ou d'un médicament, lequel produit thérapeutique ou médicament peut être éventuellement prévu pour s'éluer de la prothèse à la suite de la mise en place de la prothèse.

5. Prothèse selon l'une quelconque des précédentes revendications, dans laquelle :
l'enveloppe (22) s'étend sensiblement sur toute la longueur de la surface extérieure de l'élément tubulaire (11), et/ ou
l'élément tubulaire (11), expansible radialement, a , une fois déployé, une forme globalement cylindrique, avec une surface généralement cylindrique tournée vers l'extérieur, et l'enveloppe (22) est placée sur cette surface tournée vers l'extérieur.

6. Prothèse selon l'une quelconque des précédentes revendications, dans laquelle l'enveloppe (22) est adaptée pour épouser les contours de la paroi du vaisseau au niveau de la zone de contact entre elles suite à la mise en place de la prothèse de manière à former une étanchéité entre l'enveloppe gonflée (22) et la paroi du vaisseau et à réduire au moins une endofuite primaire entre la prothèse mise en place et le vaisseau.

7. Prothèse selon l'une quelconque des précédentes revendications, dans laquelle l'agent est adapté pour gonfler du fait de l'hydrolyse et comprend éventuellement un agent actif et cet agent actif comprend un ou plusieurs des éléments suivants :
hyaluronane, sulfates de chondroïtine, dermatane et ses sulfates, kératine et ses sulfates, et héparine, et l'agent peut être en outre de l'acide hyaluronique.

8. Prothèse selon l'une quelconque des précédentes revendications, dans laquelle l'enveloppe (22) comprend un matériau thrombogène pour stimuler l'hémostase dans la dilatation, lequel matériau thrombogène peut être présent uniquement dans la deuxième couche (21) de l'enveloppe (22) et peut comprendre un ou plusieurs des éléments suivants :
collagène, protéines de coagulation, fibrine, fibronectine, soies, polymères synthétiques, polysaccharides neutres, chitine, cellulose, amindon.

9. Prothèse selon l'une quelconque des précédentes revendications, dans laquelle l'élément tubulaire (11) a un axe central longitudinal et, par rapport à cet axe, l'enveloppe a une épaisseur radiale de x mm avant le gonflement de l'enveloppe (22) et une épaisseur radiale d'au moins 2x mm à la suite du gonflement de l'enveloppe.

10. Prothèse selon l'une quelconque des précédentes revendications, dans laquelle l'élément tubulaire (11) comprend un stent ou une greffe.

11. Prothèse selon l'une quelconque des précédentes revendications, dans laquelle :
l'agent est adapté pour permettre à l'enveloppe de gonfler lorsque l'enveloppe (22) est exposée au contact d'un fluide corporel lors de la mise en place de la prothèse (23) dans le vaisseau à partir d'un système de mise en place de prothèse ; et/ ou
lorsque la dilatation est un anévrysme, l'enveloppe (22) est adaptée pour se déployer vers l'extérieur en contact d'étanchéité avec la surface intérieure du vaisseau autour de l'anévrysme de manière à isoler le sac anévrysmal par rapport à la circulation du sang le long du vaisseau à travers le conduit intérieur de l'élément tubulaire déployé (11) ; et/ ou
la prothèse (23) est un dispositif de traitement d'anévrysme de l'aorte.

12. Système de mise en place de prothèse, comprenant la prothèse (23) selon l'une quelconque des précédentes revendications et un instrument de mise en place, dans laquelle l'instrument de mise en place comprend une gaine (40) qui est adaptée sensiblement pour couvrir la prothèse (23) pendant que la prothèse (23) est mise en place par voie endoluminale sur un site cible et sensiblement pour protéger l'enveloppe (22) de la prothèse (23) contre un contact avec un fluide corporel, la gaine (40) et la prothèse (23) étant relativement mobiles de manière à permettre à la prothèse (23) d'être déployée depuis l'intérieur de la gaine (40) au niveau du site cible pour exposer l'enveloppe de la prothèse (23) au contact d'un fluide corporel.

13. Système selon la revendication 12, dans lequel la paroi intérieure du vaisseau au niveau du site cible a des contours irréguliers et l'extérieur de l'enveloppe dilatée (23) est adaptée pour suivre les contours irréguliers afin d'établir un bon contact d'étanchéité avec la paroi intérieure du vaisseau.

14. Procédé de fabrication d'une prothèse endoluminale, comprenant les étapes consistant à :
prévoir un élément tubulaire (11) sensiblement imperméable aux fluides et expansible, ayant une extrémité proximale, une extrémité distale, un conduit intérieur, une surface extérieure et une circonférence ; et
appliquer une enveloppe (22), comprenant des première (20) et deuxième (21) couches de matériau, sur la surface extérieure de l'élément tubulaire (11) en enroulant simultanément des première (20) et deuxième (21) feuilles de matériau autour de l'élément tubulaire (11) pour former les première (20) et deuxième (21) couches respectivement et pour obtenir une enveloppe (22) qui comprend les deux feuilles intercalées l'une avec l'autre et disposées globalement en spirale ;
dans lequel la première couche (20) comprend un agent destiné à stimuler le gonflement de l'enveloppe de manière à permettre à l'enveloppe de gonfler vers l'extérieur sur l'élément tubulaire (11) lors de la mise en place de la prothèse dans un vaisseau corporel situé autour et la deuxième couche (21) comprend un matériau de remplissage d'espace, dans lequel l'élément tubulaire (11) et l'enveloppe (22) sont conçus et adaptés pour que, lors de la mise en place de la prothèse (23) dans le vaisseau corporel situé autour, l'élément tubulaire (11) se dilate radialement et l'enveloppe (22) gonfle en augmentant de volume sur l'élément tubulaire (11) de manière à presser l'extérieur de l'enveloppe gonflée (22) dans toutes les irrégularités de surface de la paroi intérieure du vaisseau corporel afin de former un bon contact d'étanchéité avec la paroi intérieure.

15. Procédé selon la revendication 14, dans lequel la prothèse fabriquée (23) est tel que revendiqué dans l'une quelconque des revendications 1 à 11.
